## Europäisches Patentamt

## European Patent Office

## Office européen des brevets

(19)

(11) Veröffentlichungsnummer: **0 129 576**
**B1**

(12) **EUROPÄISCHE PATENTSCHRIFT**

(45) Veröffentlichungstag der Patentschrift:
**02.08.89**

(21) Anmeldenummer: **84900096.3**

(22) Anmeldetag: **05.12.83**

(86) Internationale Anmeldenummer:
**PCT/HU 83/00060**

(87) Internationale Veröffentlichungsnummer:
**WO 84/02278 (21.06.84 Gazette 84/15)**

(51) Int. Cl.⁴: **A 61 M 5/315,** A 61 M 5/28

(54) **EINWEGSPRITZE AUS KUNSTSTOFF FÜR MEDIZINISCHE ZWECKE UND KOLBEN AUS KUNSTSTOFF.**

(30) Priorität: **10.12.82 HU 398982**

(43) Veröffentlichungstag der Anmeldung:
**02.01.85 Patentblatt 85/1**

(45) Bekanntmachung des Hinweises auf die Patenterteilung:
**02.08.89 Patentblatt 89/31**

(84) Benannte Vertragsstaaten:
**AT BE CH DE FR GB LI NL SE**

(56) Entgegenhaltungen:
**CH-A-366 126**
**DE-A-2 315 018**
**DE-A-2 617 083**
**DE-B-2 541 043**
**DE-U-7 540 567**
**FR-A-1 500 009**
**GB-A-1 431 989**
**US-A-3 147 753**
**US-A-3 662 753**
**US-A-3 939 833**

(73) Patentinhaber: **INNOFINANCE Altalános Innovácios Pénzintézet, Paulay Ede u. 13, H-1061 Budapest (HU)**

(72) Erfinder: **ADORJAN, András, Törcsvár u. 27/A, H-1112 Budapest (HU)**
Erfinder: **DAVID, Csaba, Arpád u. 10/B, H-1215 Budapest (HU)**

(74) Vertreter: **Finck, Dieter, Patentanwälte v. Füner, Ebbinghaus, Finck Mariahilfplatz 2 & 3, D-8000 München 90 (DE)**

EP 0 129 576 B1

## Beschreibung

Die Erfindung betrifft eine Einwegspritze aus Kunststoff für medizinische Zwecke, bestehend aus einem bodenseitig mit einem kegeligen Anschlußstück zum Aufsetzen einer Kanüle versehenen Zylinder, in dem ein mit einer Kolbenstange lösbar verbundener Kolben aus weichelastischem Material angeordnet ist, wobei der Zylinder an seinem dem Anschlußstück gegenüberliegenden Ende mit einer Verschlußkappe mit zwei bezogen auf die Achse des Zylinders koaxialen zylindrischen Bunden versehen ist, von denen der innere Bund eine die Kolbenstange gerade führende Gleitführung bildet, deren in axialer Richtung gemessene Länge mindestens gleich der größten Querschnittsabmessung der Kolbenstange ist, und von denen der äußere Bund ringwandförmig ausgebildet ist und formschlüssig mit dem Zylinder verbunden ist.

Bei einer solchen, aus der DE-A-2 315 018 bekannten Einwegspritze besteht der Spritzenzylinder aus Kunststoff. Der im Zylinder verschiebliche Kolben ist aus Gummi hergestellt, an dem eine Kolbenstange aus schlagfestem Polystyrol sitzt. Die bekannte Einwegspritze hat eine Verschlußkappe mit einem äußeren Bund, dessen Innengewinde auf ein Außengewinde am Behälterende aufschraubbar ist. Die Verschlußkappe hat weiterhin einen inneren Bund, der sich axial nach außen erstreckt, abbrechbar ist und als Führung für die Kolbenstange dient. Dabei ist die Länge der verbleibenden Führung größer als der Durchmesser der Kolbenstange.

Das bei der bekannten Einwegspritze für den Kolben benutzte Material in Form von Gummi ist für einen Wegwerfartikel sehr aufwendig, Um eine abdichtende Führung des Kolbens aus Gummi an der Zylinderinnenwand zu gewährleisten, muß ein Dichtungsmittel in Form einer Silikonlösung verwendet werden, um brauchbare Gleiteigenschaften bei geringer Startkraft zu ermöglichen. Da die bekannte Einwegspritze in ihre Einzelteile zerlegbar und aus diesen Teilen wieder zusammensetzbar ist, jedoch eine zufriedenstellende Sterilisation der einzelnen Teile nicht erreicht werden kann, ist der gewünschte Einmalgebrauch nicht gewährleistet. Es besteht vielmehr die Gefahr, daß dort, wo Spritzen Mangelware sind, durch mehrfachen Gebrauch auch bei versuchter Sterilisation zwischen den Einsätzen Viren und andere Erreger übertragen werden.

Aus der US-A-3 662 753 ist eine Spritze zum Mischen und Abgeben von zwei getrennt vorgespeicherten Substanzen bekannt, in deren Zylinder eine Verschlußkappe so eingeschoben ist, daß eine innere Randleiste elastisch in eine Aussparung zwischen Bund und Flansch der Verschlußkappe eingreift, Diese Verbindung bietet zwar einen gewissen Widerstand gegen ein Herausziehen der Verschlußkappe, Mit etwas größerem Kraftaufwand ist dieser Widerstand jedoch ohne weiteres überwindbar.

Die der Erfindung zugrundeliegende Aufgabe besteht darin, die Einwegspritze der eingangs genannten Art so auszubilden, daß ohne Beeinträchtigung der sicheren Funktion eine Demontage in Einzelteile und eine erneute Montage aus den Einzelteilen für den weiteren Gebrauch mit Sicherheit vermieden wird, also die Einwegspritze wirklich nur als Wegwerfartikel benutzt werden kann.

Diese Aufgabe wird ausgehend von der Einwegspritze der eingangs genannten Art dadurch gelöst, daß der Kolben als Formstück aus Polyethylen ausgebildet ist, daß der äußere Bund im Preßsitz an der Innenwand des Zylinders angreift, der durch eine bleibende örtliche Verformung der Pressflächen verstärkt ist und daß der Kolben als ein im wesentlichen glockenförmiger Rotationskörper mit einem an einem Kolbendeckel angeformten ringwandförmigen Kolbenmantel und mit einem im Kolbeninneren mittig angeformten Schaftteil sowie mit einer zwischen Kolbenmantel und Schaftteil vorhandenen, nach dem Kolbenboden hin offenen Aussparung ausgebildet ist, wobei der Kolbenmantel zu den in Achsrichtung des Kolbens weisenden Kolbenrändern hin sich kegelförmig ausweitende weichelastische Wandteile von stark reduzierter Wandstärke aufweist und zwischen diesen Wandteilen ein im wesentlichen formbeständiger zylindrischer Wandteil mit voller Wandstärke vorhanden ist.

Vorzugsweise hat das den Kolben bildende Polyethylen eine Shore-Härte zwischen 65 und 90.

Die erfindungsgemäße Einwegspritze hat ausgezeichnete Funktionseigenschaften, nämliche eine einwandfreie Dichtungswirkung zwischen Zylinder und Verschlußkappe sowie Kolben und Zylinderinnenwand, wo auch aufgrund des als Material verwendeten Polyethylens sehr gute Gleiteigenschaften in beiden Hubrichtungen auf der gesamten Hublänge des Kolbens bei einer geringen Startkraft gewährleistet sind, Gleichzeitig werden alle wichtigen medizinischen Anforderungen erfüllt. So ist die Einwegspritze ohne Schwierigkeiten sterilisierbar, Sie kann vollmechanisch gefertigt und in keimfreier Gasatmosphäre ohne jegliche Handarbeit steril verpackt werden, Ein Zerlegen der Einwegspritze nach dem Gebrauch und somit eine erneute Verwendung mit oder ohne Nachsterilisierung ist mit Sicherheit ausgeschlossen. Beim Aufziehen der Spritze kann eine Infizierung des Innenraums der Spritze nicht stattfinden, da bei der getroffenen Ausgestaltung eine Berührung der Kolbenstange beim Injizieren nicht stattfindet, Da auch der Herstellungsaufwand sehr gering ist, stellt die Einwegspritze einen idealen Massenartikel für den Einmalgebrauch dar, der sämtlichen Ansprüchen der europäischen Normen genügt.

Anhand von Zeichnungen wird ein Ausführungsbeispiel der Erfindung näher erläutert. Es zeigt:

Fig. 1    eine Seitenansicht der Einwegspritze teilweise im Schnitt

Fig. 2    eine Ausführungsform der Verschlußkappe im Axialschnitt und

Fig. 3 im Axialschnitt die Ausgestaltung des Kolbens.

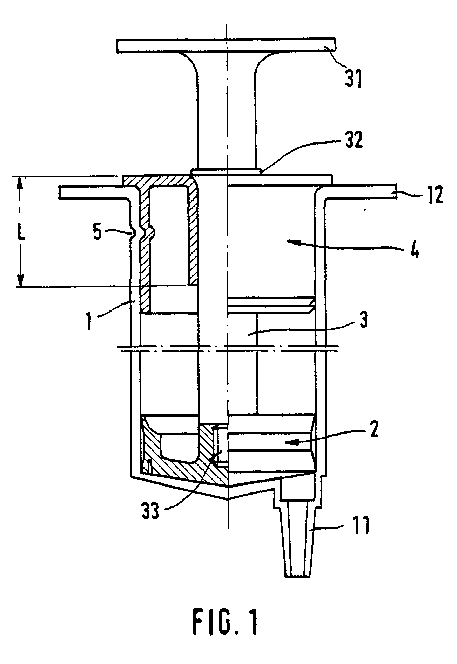

In einem im Spritzgußverfahren als Massenartikel hergestellten, im wesentlichen steifwandigen Zylinder 1, wie er in Fig. 1 gezeigt ist, ist ein wenigstens zum Teil weichelastisch gestalteter Kolben 2 dichtend geführt. Der Zylinder 1 weist an seinem bodenseitigen Ende ein Anschlußstück 11 auf, das eine durchgehende Innenbohrung besitzt und außen mit einem genormten Kegelsitz zum Aufsetzen einer nicht gezeigten Kanüle ausgebildet ist. Am offenen, in der Zeichnung oberen Zylinderende sind zwei diametral gegenüberliegende, als Fingergriff beim Injizieren dienende Nasen 12 angeformt. Der Kolben 2 ist mit einer zylindrischen Kolbenstange 3 über einen in den Kolben einschraubbaren Fortsatz 33 der Kolbenstange 3 fest, jedoch lösbar verbunden. Die Kolbenstange 3 ist im Abstand von der inneren Zylinderwand geführt. Als stabile, im Zylinderraum weitgehend verformungsarme Führung von hinreichender Länge dient eine Gleitführung 40, welche als eine in das offene Zylinderende unter haftendem Preßsitz eingesetzten Verschlußkappe 4 ausgebildet ist, wobei der Preßsitz durch eine bleibende örtliche Verformung 5 der Preßflächen verstärkt ist. Die Kolbenstange 3 ist ferner an ihrem dem Kolben 2 abgewandten Ende mit einer Griffplatte 31 zur bequemen Betätigung versehen. In der Nähe der Griffplatte 31 ist ferner an der Kolbenstange 3 ein ringförmig umlaufender Wulst 32 als Orientierungsglied und Montagehilfe angeformt.

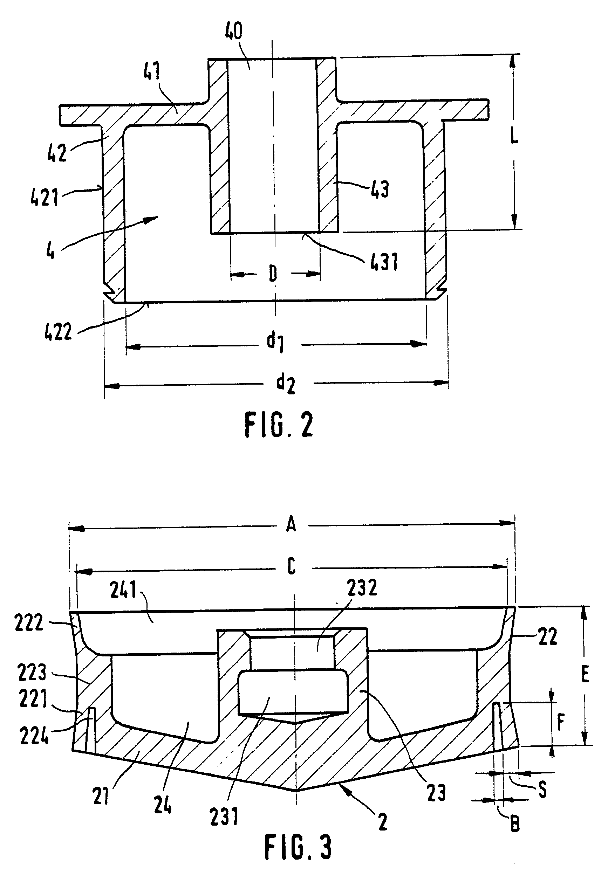

Die in Fig. 2 noch ohne Verformung 5 gezeigte Verschlußkappe 4 besteht aus einer Scheibe 41 mit zwei koaxialen, im wesentlichen zylindrischen Bunden 42, 43. Der innere Bund 43 bildet die Gleitführung 40 für die Kolbenstange. Der äußere Bund 42 ist für die kraft- und formschlüssige Verbindung zwischen Verschlußkappe 4 und Zylinder 1 ringwandförmig ausgebildet. Der innere Bund 43 kragt im Gegensatz zu der in Fig. 1 gezeigten Ausführung in axialer Richtung nach beiden Seiten hin über die Scheibe 41 aus. Kolbenseitig endet der innere Bund 43 in einer Stirnfläche 431. Wesentlich ist, daß die Länge L der Gleitführung 40 mindestens gleich der größten Querschnittsabmessung ist, also in Fig. 2 dem Durchmesser D. Der äußere Bund 42 hat eine Außenfläche 421 mit einem Durchmesser $d_2$ und eine Innenfläche mit einem Durchmesser $d_1$. Der äußere Bund 42 hat ferner eine kreisringförmige Stirnfläche 422, die als Endanschlag für den Kolben 2 in seiner eingezogenen Endstellung dient.

Der in Fig. 3 gezeigte Kolben 2 hat im wesentlichen die Gestalt eines glockenförmigen Rotationskörpers und ist aus Polyethylen mit einer Shore-Härte zwischen 65 und 90 hergestellt. An einem Kolbendeckel 21 sind ein ringwandförmiger Kolbenmantel 22 und im Kolbeninneren ein zentraler Schaftteil 23 angeformt. Zwischen dem Kolbenmantel 22 und dem Schaftteil 23 ist eine nach dem Kolbenboden hin offene ringnutförmige Aussparung 24 vorhanden.

Der Kolbenmantel 22 hat in beiden Kolbenrandbereichen sich zu den Kolbenrändern hin kegelförmig aufweitende weichelastische Wandteile 221, 222 mit stark reduzierter Wandstärke S. Zwischen diesen weichelastischen dünnen Wandteilen 221, 222 ist ein im wesentlichen formbeständiger zylindrischer Wandteil 223 mit voller Wandstärke vorhandene Im deckelseitigen Kolbenrandbereich wird der weichelastische Wandteil 221 durch eine von der Deckelseite her in den Kolbenmantel 22 eingelassene offene Ringnut 224 begrenzt. Im bodenseitigen Kolbenrandbereich ist dagegen der weichelastische Wandteil 222 durch eine abgestufte Aufweitung 241 der ringnutförmigen Aussparung 24 ausgebildet.

Bei einem Ausführungsbeispiel der Einwegspritze beträgt die stark reduzierte Wandstärke 5 der weichelastischen Wandteile 221, 222 jeweils an den Kolbenrändern gemessen 0,3 bis 0,5 mm Die in axialer Richtung gemessene Höhe E des Kolbenmantels 22 liegt bei 5 bis 7 mm. Die in der Kolbendeckelebene gemessene Breite B der in den Kolbenmantel 22 deckelseitig eingelassenen offenen Ringnut 224 liegt zwischen 0,3 bis 0,5 mm während die Tiefe F der Ringnut 224 je nach Kolbengröße 2 bis 2,5 mm beträgt. Der im wesentlichen formbeständige zylindrische Wandteil 223 hat einen Durchmesser C, der höchstens gleich dem Innendurchmesser des den Kolben 2 dichtend führenden Zylinders 1 ist Die größten Durchmesser A der sich kegelförmig aufweitenden weichelastischen Wandteile 221, 222 sind um 0,2 bis 0,6 mm größer als der Innendurchmesser des Zylinders 1.

In dem im Kolbeninneren mittig angeformten Schaftteil 23 kann ein Innengewinde zur festen, jedoch lösbaren Verbindung des Kolbens 2 mit seiner Kolbenstange 3 vorhanden sein, wie es der Fig. 1 entnehmbar ist. Fig. 3 zeigt eine Ausführung, bei welcher im Schaftteil 23 ein bodenseitig durch eine Bohrung 232 hindurch zugänglicher zylindrischer Hohlraum 231 vorgesehen ist. Der Durchmesser des Hohlraumes 231 ist größer gewählt als derjenige der Bohrung 232, so daß ein entsprechend mit Übermaß gegenüber dem Bohrungsdurchmesser tolerierter Fortsatz 33 der Kolbenstange 3 durch die Bohrung 232 hindurch mit einer bestimmten Kraft für ein Einschnappen in den Hohlraum 231 eingeführt werden kann, wodurch eine hinreichend feste, jedoch lösbare Verbindung zwischen Kolben 2 und Kolbenstange 3 erhalten wird.

**Patentansprüche**

1. Einwegspritze aus Kunststoff für medizinische Zwecke, bestehend aus einem bodenseitig mit einem kegeligen Anschlußstück (11) zum Aufsetzen einer Kanüle versehenen Zylinder (1), in dem ein mit einer Kolbenstange (3) lösbar verbundener Kolben (2) aus weichelastischem Material angeordnet ist, wobei der Zylinder (1) an seinem dem Anschlußstück (11) gegenüberliegenden Ende mit einer Verschlußkappe (4) mit zwei bezogen auf die Achse des Zylinders (1) koaxialen zylindrischen Bunden (42, 43) versehen ist, von denen der innere Bund (43) eine die Kolbenstange (3) gerade führende Gleitführung (40) bildet, deren in axialer Richtung gemessene Länge (L) mindestens gleich der größten Querschnittsabmessung (D) der Kolbenstange (3) ist, und von denen der äußere Bund (42) ringwandförmig ausgebildet ist und formschlüssig mit dem Zylinder (1) verbunden ist, dadurch gekennzeichnet, daß der Kolben (2) als Formstück aus Polyethylen ausgebildet ist, daß der äußere Bund (42) der Verschlußkappe (4) im Preßsitz an der Innenwand des Zylinders (1) angreift, der durch eine bleibende örtliche Verformung (5) der Preßflächen verstärkt ist) und daß der Kolben (2) als ein im wesentlichen glockenförmiger Rotationskörper mit einem an seinem Kolbendeckel (21) angeformten ringwandförmigen Kolbenmantel (22) und mit einem im Kolbeninneren mittig angeformten Schaftteil (23) sowie mit einer zwischen Kolbenmantel (22) und Schaftteil (23) vorhandenen, nach dem Kolbenboden hin offenen Aussparung (24) ausgebildet ist, wobei der Kolbenmantel (22) zu den in Achsrichtung des Kolbens (2) weisenden Kolbenrändern hin sich kegelförmig ausweitende weichelastische Wandteile (221, 222) von stark reduzierter Wandstärke aufweist und zwischen diesen Wandteilen (221, 222) ein im wesentlichen formbeständiger zylindrischer Wandteil (223) mit voller Wandstärke vorhanden ist.

2. Einwegspritze nach Anspruch 1, dadurch gekennzeichnet, daß das den Kolben (2) bildende Polyethylen eine Shore-Härte zwischen 65 und 90 hat.

## Claims

1. A disposable synthetic plastics syringe for medicinal purposes, consisting of a barrel (1) provided at one end with a conical connector (11) on which it is possible to fit a cannula, a piston (2) which is separably connected to a piston rod (3) and which consists of softly-elastic material being disposed inside the barrel (1) which has, at its end opposite the connector (11), with a closure cap (4) with two cylindrical shoulders (42, 43) which are coaxial with the barrel (1) and of which the inner collar (43) constitutes a sliding guide (40) which guides the piston rod (3) to keep it straight, the length (L) of the sliding guide (40) measured in an axial direction, being at least equal to the greatest croßsectional dimension (D) of the piston rod (3) and of which the outer shoulder (42) is constructed as an annular wall which is form-lockingly connected to the barrel (1), characterised in that the piston (2) is moulded from polyethylene and in that the outer shoulder (42) of the closure cap (4) engages with a press fit against the inside wall of the barrel (1) which is strengthened by a lasting localised deformation (5) in the surfaces which are press-fitted together, and in that the piston (2) is constructed as a substantially bell-shaped body of rotation with an annular-wall-shaped piston shell (22) integrally moulded on its piston cover (21), and with a stein part (23) integrally moulded in the centre, inside the piston, and with, between the piston shell (22) and the stem part (23), a recess (24) which is open towards the end of the piston, the piston shell (22) comprising softly-elastic will parts (221, 222) of greatly reduced wall thickness which widens out conically towards the piston edges which point in the axial direction of the piston (2), the full wall thickness being provided between the wall parts (221, 222) and a substantially stable cylindrical wall part (223).

2. A disposable syringe according to Claim 1, characterised in that the polyethylene used for the piston (2) has a Shore hardness of between 65 and 90.

## Revendications

1. Seringue jetable en plastique à usage médical, constituée d'un cylindre (1) qui, côté fond, est muni d'une pièce de raccordement (11) conique pour y placer une canule, et dans lequel est disposé un piston (2), en matériau à élasticité souple, relié, de façon amovible, avec une tige de piston (3), le cylindre (1) étant muni, à son extrémité opposée à la pièce de raccordement (11), d'un capuchon d'obturation (4) qui présente deux couronnes (42, 43) cylindriques, coaxiales à l'axe du cylindre (1), dont la couronne intérieure (43) forme un guidage coulissant (40) qui guide avec précision la tige de piston (3) et dont la longueur (L), mesurée en direction axiale, est au moins égale à la plus grande dimension en section (D) de la tige de piston (3), et dont la couronne extérieure (42) est conçue sous forme de paroi annulaire et est reliée, de par la forme, avec le cylindre (1), caractérisée en ce que le piston (2) est conçu comme pièce de moulage en polyéthylène; en ce que la couronne extérieure (42) du capuchon d'obturation (4) s'applique, à ajustement serré, contre la paroi intérieure du cylindre (1) ajustement qui est renforcé par une déformation locale permanente (5) des surfaces ajustées; et en ce que le piston (2) est conçu sous forme d'un corps de révolution sensiblement en forme de cloche présentant une surface latérale (22) en forme de paroi annulaire venue de moulage sur son couvercle (21), ainsi qu'une partie formant fût (23) venue de moulage dans l'axe dans l'intérieur du piston, ainsi qu'un évidement (24) existant entre la surface latérale (22) du piston et de la partie format fût (23) et ouvert en direction du fond du piston, la surface latérale (22) du piston présentant dos parties de paroi (221, 222), d'épaisseur de paroi fortement réduite, à élasticité souple, allant en s'élargissant en forme de cône en direction des bords du piston orientés dans la direction axiale du piston (2), et entre ces parties de paroi (221, 222), existant une partie de paroi cylindrique (223), d'épaisseur de paroi totale, sensiblement rigide de forme.

2. Seringue jetable selon la revendication 1, caractérisée en ce que le polyéthylène qui forme le piston (2) présente une dureté Shore comprise entre 65 et 90.

FIG. 1

FIG. 2

FIG. 3